# EUROPEAN PATENT APPLICATION

(11) **EP 2 977 008 A1**
(43) Date of publication of application: **27.01.2016**
(21) Application number: 14770798.8
(22) Date of filing: 18.03.2014
(51) Int. Cl.: A61B 6/00, A61N 5/00, G09B 23/28

(54) **METHOD, APPARATUS, AND SYSTEM FOR MANUFACTURING PHANTOM CUSTOMIZED TO PATIENT**

(30) Priority: 19.03.2013 KR 20130029248
(71) Applicant: Samsung Life Public Welfare Foundation, Seoul 140-893 (KR)
(72) Inventor: JU, Sang Gyu, Seoul 135-230 (KR)
(74) Representative: Merryweather, Colin Henry
(86) International application number: PCT/KR2014/002272
(87) International publication number: WO 2014/148794

(57) **Abstract**

An apparatus and method for generating print data about a phantom customized to a patent, the apparatus receive medical images including anatomical information of a patient's body and treatment plan information about a dose distribution of a radiation to be irradiated to a treatment area, reconstructs a three-dimensional phantom image in which internal and external structures of the patient's body are modeled which are based on the anatomical information and in which a space for a dosimeter is modeled which is to be inserted into the internal and external structures so as to verify the dose distribution according to the treatment plan information, and generates print data for three-dimensional printing, which is used for manufacturing the phantom customized to the patient on the basis of the reconstructed three-dimensional phantom image.

## Description

### TECHNICAL FIELD

The present invention relates to a method, an apparatus, and a system for manufacturing a phantom customized to a patient, and more particularly, to a method, an apparatus, and a system for manufacturing a phantom customized to a patient by using a three-dimensional printing device.

### BACKGROUND ART

Recently, with the increase in aging population due to an increase in average life and the development of an early diagnosis technology, the number of cancer patients has tended to abruptly increase. According to the Ministry for Health, Welfare and Family Affairs in 2008, when people live up to an average life, statistics that one in three Koreans has cancer have been released. A radiation treatment is one of three cancer treatment methods together with surgery and an anticancer therapy and has gradually increased its role. An object of the radiation treatment is to minimize damage to surrounding normal organs and necrose a tumor or suppress growth of the tumor by concentrating a high dose radiation on the tumor. Recently, in order to achieve the object, a high level treatment skill such as an intensity modulated radiation therapy (IMRT) has been introduced which modulates an irradiated radiation intensity to concentrate the radiation on the tumor. The high level radiation treatment skill has many advantages but has limitations in that a treatment time is long, an irradiation process is complicated, and the possibility of occurrence of radiation accident is relatively high compared to a general treatment due to a radiation dosage that is 2 to 5 times greater than the general treatment. Therefore, in order to prevent the radiation accident and verify an accurate radiation dose, it is recommended that a strict management is performed before the radiation treatment. In addition, in order to design the accurate radiation dose, a complex calculation algorithm has been introduced to a radiation treatment planning system, but a reasonable phantom and procedure that verifies the accuracy of the radiation dose under the same condition as the patient has not been established.

### DETAILED DESCRIPTION OF THE INVENTION

### TECHNICAL PROBLEM

Provided are a method, an apparatus, and a system for manufacturing a phantom customized to a patient by using a three-dimensional printing device. A technical problem to be solved by the present embodiment is not limited technical problems as described above, and other technical problems may exist.

### TECHNICAL SOLUTION

According to an aspect, an apparatus for generating print data about a phantom customized to a patent includes: a data reception unit that receives medical images including anatomical information of a patient's body and treatment plan information about a dose distribution of a radiation to be irradiated to a treatment area; an image reconstruction unit that reconstructs a three-dimensional phantom image in which internal and external structures of the patient's body are modeled which are based on the anatomical information and in which a space for a dosimeter is modeled which is to be inserted into the internal and external structures so as to verify the dose distribution according to the treatment plan information; and a print data generation unit that generates print data for three-dimensional printing, which is used for manufacturing the phantom customized to the patient on the basis of the reconstructed three-dimensional phantom image.

In addition, the image reconstruction unit reconstructs the three dimensional phantom image by modeling densities of materials constituting the internal structure by using pixel values of the received medical images.

In addition, the reconstructed three-dimensional phantom image includes information about an insertion path or cut plane for inserting the dosimeter into the phantom customized to the patient.

In addition, the reconstructed three-dimensional phantom image includes information about a mark which guides at least one of an irradiation position and a position of the dosimeter, which is used for verifying the dose distribution in the phantom customized to the patient.

In addition, the apparatus for generating print data about the phantom customized to the patent further includes a verification unit that verifies accuracies of the phantom customized to the patient and the treatment plan information by comparing data about the dose distribution measured in the inserted dosimeter with the received treatment plan information when the radiation is irradiated to the phantom customized to the patient according to the treatment plan information after the phantom customized to the patient is manufactured on the basis of the generated print data, and the dosimeter is inserted into the phantom customized to the patient.

In addition, the image reconstruction unit corrects the reconstructed three-dimensional phantom image according to the verified accuracies, and the print data generation unit regenerates the print data for three-dimensional printing on the basis of the corrected three-dimensional phantom image.

In addition, the apparatus for generating print data about the phantom customized to the patent further includes a user interface unit that receives, from a user, editing information about at least one of the modeled internal and external structures of the patient's body and the modeled space for the dosimeter, wherein the image reconstruction unit reconstructs the three-dimensional phantom image by modeling the at least one of the internal and external structures and the space according to the received editing information.

According to another aspect, a method of manufacturing a phantom customized to a patent includes: receiving medical images including anatomical information of a patient's body and treatment plan information about a dose distribution of a radiation to be irradiated to a treatment area; reconstructing a three-dimensional phantom image in which internal and external structures of the patient's body are modeled which are based on the anatomical information and in which a space for a dosimeter 14 is modeled which is to be inserted into the internal and external structures so as to verify the dose distribution according to the treatment plan information; generating print data for three-dimensional printing, which is used for manufacturing the phantom customized to the patient on the basis of the reconstructed three-dimensional phantom image; and manufacturing the phantom customized to the patient on the basis of the generated print data in a three-dimensional printing device.

In addition, the manufacturing manufactures the phantom customized to the patient by performing printing such that densities of materials constituting an inside of the phantom customized to the patient are different from one another according to pixels values of the received medical images.

In addition, the measuring manufactures the phantom customized to the patient on the basis of the generated print data such that a space for the dosimeter is emptied inside of the phantom customized to the patient and a cut plane for inserting the dosimeter has a cut state.

According to another aspect, a system for manufacturing a phantom customized to a patent includes: a computing device that reconstructs a three-dimensional phantom image in which internal and external structures of a patient's body are modeled which are based on anatomical information included in medical images with respect to the patient's body and in which a space for a dosimeter is modeled which is to be inserted into the internal and external structures so as to verify a dose distribution according to treatment plan information about the dose distribution of a radiation to be irradiated to a treatment area, and generates print data for three-dimensional printing, which is used for manufacturing the phantom customized to the patient on the basis of the reconstructed three-dimensional phantom image; and a three-dimensional printing device that manufactures the phantom customized to the patient on the basis of the generated print data.

In addition, the computing device reconstructs the three-dimensional phantom image by modeling densities of materials constituting the internal structure by using pixel values of the received medical images.

In addition, the reconstructed three-dimensional phantom image includes information about an insertion path or cut plane for inserting the dosimeter into the phantom customized to the patient.

In addition, after the phantom customized to the patient is manufactured by the three-dimensional printing device, and the dosimeter is inserted into the phantom customized to the patient, when the radiation is irradiated to the phantom customized to the patient according to the treatment plan information, the computing device verifies accuracies of the phantom customized to the patient and the treatment plan information by comparing data about the dose distribution measured in the inserted dosimeter with the received treatment plan information.

In addition, the computing device corrects the reconstructed three-dimensional phantom image according to the verified accuracies and regenerates the print data for three-dimensional printing on the basis of the corrected three-dimensional phantom image, and the three-dimensional printing device remanufactures the phantom customized to the patient on the basis of the regenerated print data.

### ADVANTAGEOUS EFFECTS OF THE INVENTION

According to the descriptions above, since phantom may be manufactured under the same condition as an actual body form of a patient, the phantom customized to the patient may be easily manufactured. In addition, accuracy of a verification of a radiation distribution or an ultrasonic distribution may be improved in a desired interest area by using the phantom customized to the patient before a radiation treatment or an ultrasonic treatment

### DESCRIPTION OF THE DRAWINGS

Fig. 1 is a block diagram for a system for manufacturing a phantom customized to a patient, according to an embodiment of the present invention.
Fig. 2 is a detailed block diagram of a computing device in a system for manufacturing a phantom customized to a patient, according to an embodiment of the present invention.
Fig. 3 is a diagram illustrating medical images including a contour of a patient's body or anatomical information, according to an embodiment of the present invention.
Fig. 4 is a diagram illustrating treatment plan information according to an embodiment of the present invention.
Fig. 5 is a diagram of a three-dimensional phantom image reconstructed in an image reconstruction unit, according to an embodiment of the present invention.
Fig. 6 is a cross-sectional view of a phantom customized to a patient according to an embodiment of the present invention.
Fig. 7 is a side sectional view of a phantom customized to a patient according to an embodiment of the present invention.
Fig. 8 is a flowchart of a method of manufacturing a phantom customized to a patient, according to an embodiment of the present invention.

### BEST MODE

An apparatus for generating print data about a phantom customized to a patent includes: a data reception unit that receives medical images including anatomical information of a patient's body and treatment plan information about a dose distribution of a radiation to be irradiated to a treatment area; an image reconstruction unit that reconstructs a three-dimensional phantom image in which internal and external structures of the patient's body are modeled which are based on the anatomical information and in which a space for a dosimeter is modeled which is to be inserted into the internal and external structures so as to verify the dose distribution according to the treatment plan information; and a print data generation unit that generates print data for three-dimensional printing, which is used for manufacturing the phantom customized to the patient on the basis of the reconstructed three-dimensional phantom image.

A method of manufacturing a phantom customized to a patent includes: receiving medical images including anatomical information of a patient's body and treatment plan information about a dose distribution of a radiation to be irradiated to a treatment area; reconstructing a three-dimensional phantom image in which internal and external structures of the patient's body are modeled which are based on the anatomical information and in which a space for a dosimeter 14 is modeled which is to be inserted into the internal and external structures so as to verify the dose distribution according to the treatment plan information; generating print data for three-dimensional printing, which is used for manufacturing the phantom customized to the patient on the basis of the reconstructed three-dimensional phantom image; and manufacturing the phantom customized to the patient on the basis of the generated print data in a three-dimensional printing device.

A system for manufacturing a phantom customized to a patent includes: a computing device that reconstructs a three-dimensional phantom image in which internal and external structures of a patient's body are modeled which are based on anatomical information included in medical images with respect to the patient's body and in which a space for a dosimeter is modeled which is to be inserted into the internal and external structures so as to verify a dose distribution according to treatment plan information about the dose distribution of a radiation to be irradiated to a treatment area, and generates print data for three-dimensional printing, which is used for manufacturing the phantom customized to the patient on the basis of the reconstructed three-dimensional phantom image; and a three-dimensional printing device that manufactures the phantom customized to the patient on the basis of the generated print data.

### MODE OF THE INVENTION

Hereinafter, embodiments of the present invention will be described. The following deceptions and the accompanying drawings are provided for understanding operations according to the present embodiment, and portions that can be easily carried out by those skilled in the art may be omitted.

In addition, the specification and the accompanying drawings are not provided to limit the present embodiment, the scope of the present embodiment should be defined by the following claims. It should be understood, however, that it is not intended to limit the invention to the particular form disclosed herein, but rather, the invention is to cover all modifications, equivalents, and alternatives falling within the spirit and scope of the invention as defined by the claims.

Hereinafter, the present embodiment will be described in more detail with reference to the accompanying drawings.

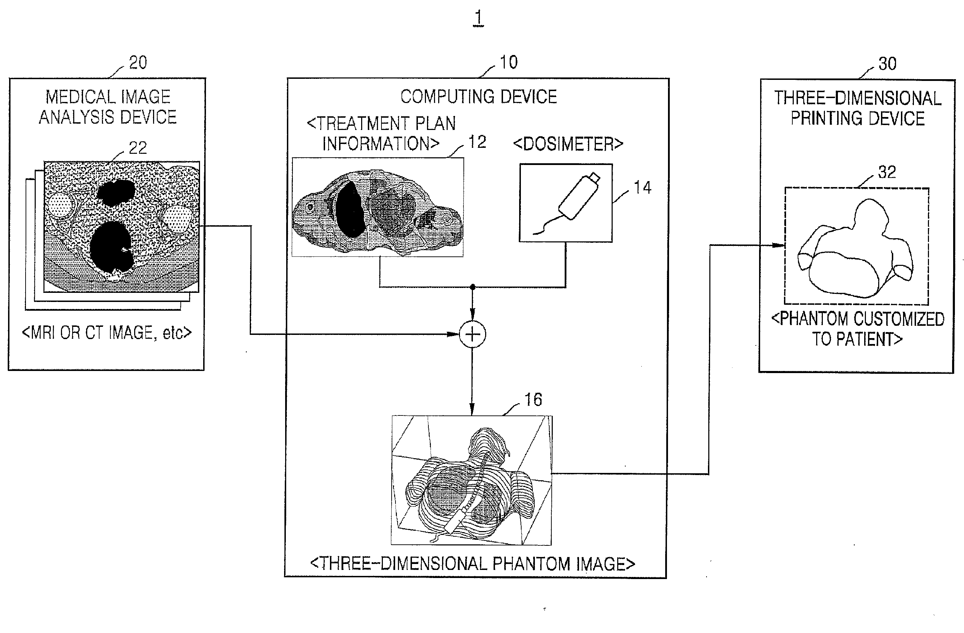

Fig. 1 is a block diagram illustrating a system 1 for manufacturing a phantom customized to a patient, according to an embodiment of the present invention. Referring to Fig. 1, the system 1 for manufacturing the phantom customized to the patient includes a computing device 10, a medical image analysis device 20, and a three-dimensional printing device 30.

The system 1 for manufacturing the phantom customized to the patient according to the present embodiment is a system for manufacturing a phantom for verifying a distribution of a radiation or a distribution of an ultrasonic wave, which is to be irradiated to the patient before a medical procedure such as a radiation treatment or an ultrasonic treatment.

Hereinafter, for convenience in description, the case of manufacturing a phantom for the radiation treatment will be described as an example, but it will be understood by those of ordinary skill in the art that the present embodiment may be equally applied to even the case of manufacturing a phantom for a different kind of treatment such as an ultrasonic treatment in addition to the radiation treatment.

The radiation treatment is one of three cancer treatment methods together with surgery and an anticancer therapy and has gradually increased its role. An object of the radiation treatment is to minimize a hazard to surrounding normal organs and necrose a tumor or suppress growth of the tumor by concentrating a high dose radiation on the tumor. Recently, in order to achieve the object, a high level treatment skill, such as an intensity modulated radiation therapy (IMRT), has been introduced which modulates an irradiated radiation intensity to concentrate the radiation on the tumor.

The high level radiation treatment skill has many advantages but has limitations in that a treatment time is long, an irradiation process is complicated, and the possibility of occurrence of radiation accident is relatively high compared to a general treatment due to a radiation dosage that is 2 to 5 times greater than the general treatment. Therefore, in order to prevent the radiation accident and verify a precise radiation dose, various radiation phantoms have been used before the radiation treatment.

In order to accurately evaluate a distribution dose of a radiation according to a radiation treatment plan, an in-vivo dosimetry that directly inserts a dosimeter into a patient's body to measure the distribution dose of the radiation is most accurate, but in-vivo dosimetry is realistically impossible.

In order to replace the in-vivo dosimetry, as described above, an existing method of verifying the radiation dose has used a method of indirectly verifying the radiation dose by irradiating an radiation planed through a radiation treatment plan process to a formulaic phantom mostly having a circular or hexahedral shape, and then inserting a dosimeter into a set position.

However, conventionally, in a process of manufacturing the phantom, the phantom has been manufactured through a cutting and manufacturing method using milling, a drill, or the like, and it has been difficult to manufacture a phantom customized to an actual body form of the patient. Therefore, since the existing formulaic phantom has a geometrical structure that is different from the body form of the patient, it has been difficult to verify the dose distribution of the radiation that matches an actual patient or verify the radiation dose at a desired position.

The system 1 for manufacturing the phantom customized to the patient according to the present embodiment is a system for designing a three-dimensional phantom image 16 by using treatment plan information 12, information of a dosimeter 14, or medical images 22 obtained from the medical image analysis device 20 (for example, a magnetic resonance imaging (MRI) device, a computed tomography (CT) device, or an ultrasonic device), and manufacturing a phantom 32 customized to a patient by using the three-dimensional printing device 30.

Therefore, the phantom 32 customized to the patient, which is more similar as the actual body form of the patient, may be manufactured by using the system 1 for manufacturing the phantom customized to the patient, and the dose distribution of the radiation according to the treatment plan information 12 may also be more accurately verified through the phantom 32 customized to the patient.

The dosimeter 14, which is to be used in the system 1 for manufacturing the phantom customized to the patient, includes all of commonly known measuring instruments capable of measuring the dose distribution, such as an ionization chamber, a film, and a multi-channel detector array, which is capable of measuring an absolute dose.

Referring to Fig. 1, the medical image analysis device 20 generally means a device that captures the medical images 22 with respect to the patient's body, and stores and analyzes the captured medical images 22. More specifically, as described above, the medical image analysis device 20 includes commonly known devices for obtaining the various medical images 22, such as the magnetic resonance imaging (MRI) device, the computed tomography (CT) device, the ultrasonic device, and an X-ray device. That is, since it is obvious to those skilled in the art that the medical image analysis device 20 is a device for obtaining the medical images 22 such as an MRI image, a CT image, an ultrasonic image, and an X-ray image, and analyzing the medical images 22, a detailed description of the medical image analysis device 20 will be omitted.

The computing device 10 models (reconstructs) the three-dimensional phantom image 16 by using the medical images 22 obtained from the medical image analysis device 20, by using the treatment plan information 12 about an irradiation distribution of a radiation, an ultrasonic wave, or the like, which is to be irradiated to a treatment area, and by using information about a type or a shape of the dosimeter 14 that is to be inserted into the phantom 32 customized to patient so as to verify the irradiation distribution of the radiation, the ultrasonic wave, or the like according to the treatment plan information 12. In addition, the computing device 20 generates print data for three-dimensional printing on the basis of the three-dimensional phantom image 16.

Here, since the three-dimensional phantom image 16 is modeled (reconstructed) by using at least the medical images 22 with respect to the patient, the print data for three-dimensional printing may be print data on which the body form of the patient or an anatomical structure of the patient is almost identically reflected.

The three-dimensional printing device 30 is a device that includes a commonly known three-dimensional printer, and more particularly, means a device that prints a desired structure three-dimensionally by using at least one of a method of manufacturing a structure by accumulating plastic or a photo-curable material according to the received print data or a method of manufacturing a structure by grinding the plastic or the photo-curable material according to the received print data.

The three-dimensional printing device 30 according to the present embodiment manufactures the phantom 32 customized to the patient on the basis of the print data for three-dimensional printing, which is generated in the computing device 30.

As described above, the three-dimensional printing device 30 may print the phantom 32 customized to the patient according to the print data on the basis of the three-dimensional phantom image 16, on which the body form of the patient or the anatomical structure of the patient is almost identically reflected, may manufacture the phantom 32 customized to patient, which more accurately matches the body form of the patient, and may more accurately verify the distribution dose of the radiation according to the treatment plan information 12 through the phantom 32 customized to patient.

Hereinafter, an operation and a function of the computing device 10 will be described in more detail which generates the print data for three-dimensional printing before printing the phantom 32 customized to the patient.

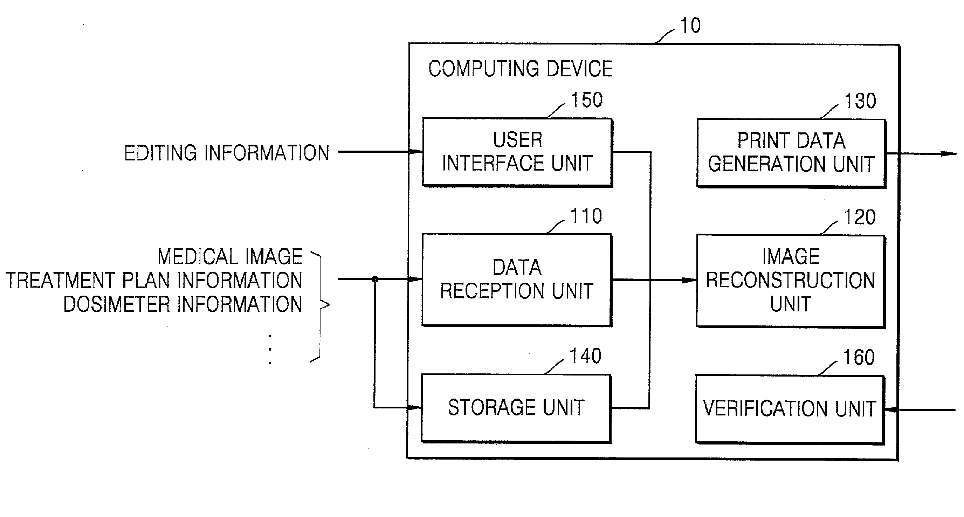

Fig. 2 is a detailed block diagram of the computing device 10 in the system 1 for manufacturing the phantom customized to the patient, according to an embodiment of the present invention.

Referring to Fig. 2, the computing device 10 includes a data reception unit 110, an image reconstruction unit 120, a print data generation unit 130, a storage unit, 140, a user interface unit 150, and a verification unit 160. In the computing device 10, only configurations retaliating to the present embodiment are illustrated in Fig. 2 for preventing characteristics of the present embodiment from becoming ambiguous, but it will be understood by those of ordinary skill in the art that other common configurations may be further included in addition to the configurations illustrated in Fig. 2.

Here, some configurations of the computing device 10 may be implemented by using at least one processor generally used. In particular, the image reconstruction unit 120, the print data generation unit 130, and the verification unit 160 of the computing device 10 may be implemented by using at least one processor generally used.

The data reception unit 110 receives the medical images (22 of Fig. 1) that include the anatomical information of the patient's body and the treatment plan information (12 of Fig. 1) about the dose distribution of the radiation that is to be irradiated to the treatment area.

The medical images 22 are data received from the image analysis device (20 of Fig. 1) described above and includes an MRI image, a CT image, an ultrasonic image, or the like that includes the contour of the patient's body or the anatomical information of the patient's body.

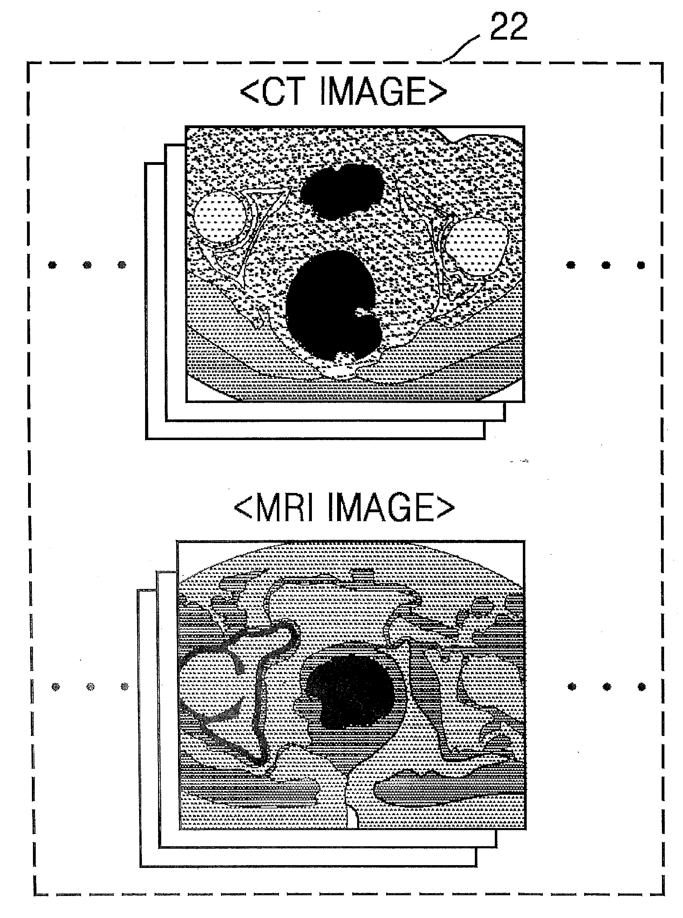

Fig. 3 is a diagram illustrating the medical images 22 including the contour of the patient's body or the anatomical information, according to an embodiment of the present invention. Referring to Fig. 3, the medical images 22 may correspond to a plurality of two-dimensional CT images or a plurality of MRI images, which are obtained by photographing sections of the patient's body. In addition, although not illustrated in Fig. 3, the medical images 22 received from the medical image analysis device 20 may correspond to a three dimensional CT image or MRI image.

Generally, the contour of the patient's body and the anatomical information are displayed on the medical images 22 such as the CT image or the MRI image so as to be distinguished in different pixel values. When the pixel values are used, the anatomical information may be distinguished, such as a bone or an organ in the patient's body.

The treatment plan information 12 is information about the dose distribution of the radiation that is to be irradiated to the treatment area in the patient's body and means the result obtained by pre-simulating the radiation doses irradiated to the treatment area are and surroundings of the treatment area.

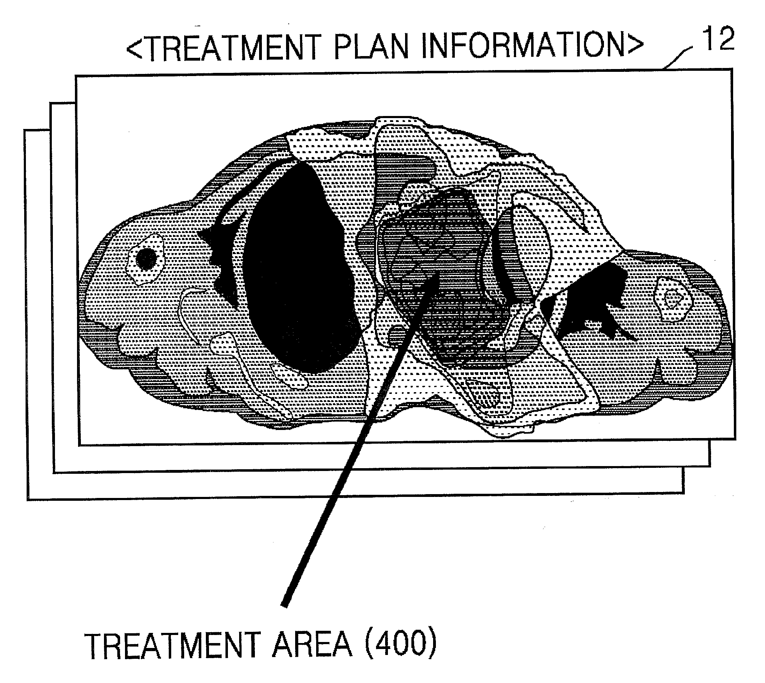

Fig. 4 is a diagram illustrating the treatment plan information 12 according to an embodiment of the present invention. Referring to Fig. 4, the treatment plan information 12 is information in which the dose distributions of the radiations are simulated which are irradiated to a treatment area 400 and surroundings of the treatment area 400 when the treatment area 400 is designated on the medical images (22 of Fig. 1) such as the CT image or the MRI image, and a radiation treatment is performed on the designated treatment area 400.

The dose distributions of the radiations irradiated to the treatment area 400 and the surroundings of the treatment area 400 are illustrated in Fig. 4 as being distinguished in different colors. For example, the radiation is concentrated on and irradiated to the treatment area 400 and is displayed in a red color region having a high dose distribution.

As will be described below, since the phantom (32 of Fig. 1) customized to the patient is capable of being utilized as a use for verifying and measuring the dose distributions of the radiations irradiated to the treatment area 400 and surroundings of the treatment area 400, a space, into which the dosimeter (14 of FIG 1) for measuring the radiation dose is to be inserted, is needed at a position of the treatment area 400 of the phantom 32 customized to the patient, which is to be manufactured later.

Referring again to Fig. 2, the image reconstruction unit 120 reconstructs the three-dimensional phantom image 16 in which internal and external structures of the patient's body are modeled which are based on the anatomical information included in the medical images 22 and in which the space for the dosimeter 14 is modeled which is to be inserted into the internal and external structures so as to verify the dose distribution according to the treatment plan information 12.

More specifically, first, the image reconstruction unit 120 models the internal and external structures of the patient's body three-dimensionally by segmenting the anatomical information included in the medical images 22.

If a description is given with reference to Fig. 3, the medical images 22 such as the plurality of CT images or the plurality of MRI images correspond to images with respect to sections of the patient's body, and the image reconstruction unit 120 reconstructs the internal and external structures of the patient's body three-dimensionally by matching the respective images. A process of modeling (reconstructing) the three-dimensional image by using the plurality of the CT images or the plurality of MRI images is obvious to those of ordinary skill in the art, and a detailed description thereof will be omitted.

In the process of modeling the internal and external structures of the patient's body on the basis of the anatomical information, the image reconstruction unit 120 may model the three-dimensional phantom image 16 by calculating physical densities or electron densities of materials constituting the internal structure of the patient's body by using the pixel values included in the medical images 22.

For example, since densities of human body constituting materials such as a subcutaneous fatty tissue, a bone, and an organ are different from one another in the patient's body, the human body constituting materials are displayed so as to have other pixel values in the medical images 22. This is also a principle of generating the medical images 22 such as the MRI image or the CT image.

Therefore, the image reconstruction unit 120 reconstructs the three-dimensional phantom image 16 such that densities of structures inside the three-dimensional phantom image 16 correspond to the pixel values included in the medical images 22. In addition, the image reconstruction unit 120 may reconstruct the three-dimensional phantom image 16 such that the structures inside the three-dimensional phantom image 16 have different colors, respectively.

Next, the image reconstruction unit 120 reconstructs the three-dimensional phantom image 16 such that information about an insertion path or cut plane for inserting the dosimeter 14 is included in the three-dimensional phantom image 16. The insertion path for inserting the dosimeter 14 may be reconstructed in an intaglio shape in the three-dimensional phantom image 16.

In addition, the image reconstruction unit 120 reconstructs the three-dimensional phantom image 16 such that information about a mark is included in the three-dimensional phantom image 16, the mark guiding an irradiation position of the radiation or a position of the dosimeter 14, which is used to verify the dose distribution later.

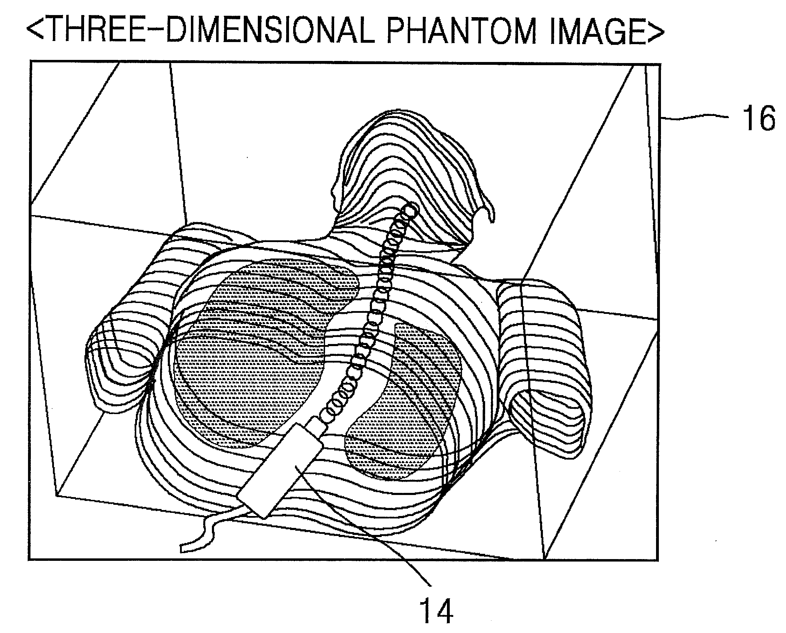

Fig. 5 is a diagram of the three-dimensional phantom image 16 reconstructed in the image reconstruction unit 120, according to an exemplary embodiment of the present invention.

Referring to Fig. 5, in the three-dimensional phantom image 16, the internal and external structures of the patient's body are modeled, which are based on the anatomical information included in the medical images (22 of Fig. 1). In addition, in three-dimensional phantom image 16, a position of the dosimeter 14 and a space for the dosimeter 14 are modeled which is to be inserted into the phantom (32 of Fig. 1) customized to the patient later.

As described above, since the three-dimensional phantom image 16 is an image that is reconstructed on the basis of the medical images 22 of the patient, the three-dimensional phantom image 16 reflects the body form of the patient or the anatomical structure of the patient almost equally.

Referring again to Fig. 2, the print data generation unit 130 generates the print data for three-dimensional printing, which is used for manufacturing the phantom 32 customized to the patent on the basis of the three-dimensional phantom image 16 reconstructed in the image reconstruction unit 120. As described above, the print data is data having a format that is required for printing a three-dimensional structure in the three-dimensional printing device (30 of Fig. 1). That is, the print data generation unit 130 converts image data of the three-dimensional phantom image 16 into print data having a format that is construable in the three-dimensional printing device 30. Since the print data having the converted format is obvious to those of ordinary skill in the art relating to the three-dimensional printing device 30, a detailed description thereof will be omitted.

The three-dimensional printing device 30 receives the print data for three-dimensional printing generated in the print data generation unit 130 and manufactures the phantom 32 customized to the patient on the basis of the print data for three-dimensional printing.

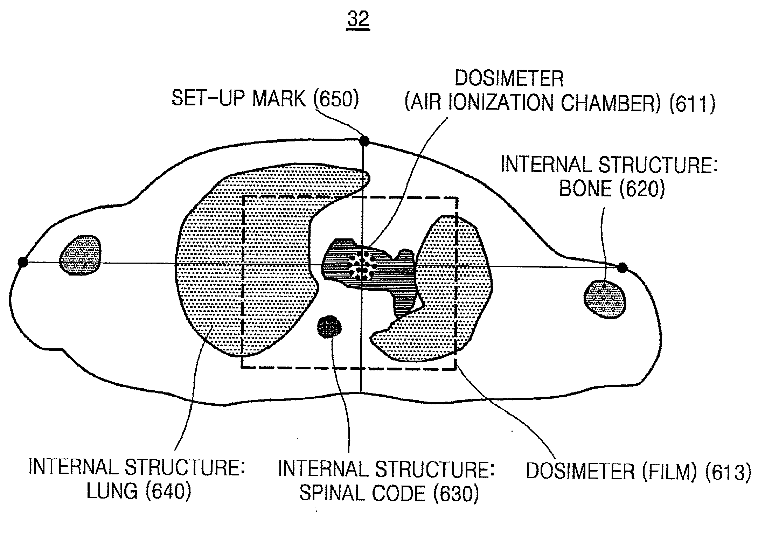

Fig. 6 is a cross-sectional view of the phantom 32 customized to the patient according to an exemplary embodiment of the present invention. Referring to Fig. 6, the phantom 32 customized to the patient is a structure manufactured by accumulating plastic or a photo-cured material or a structure manufactured by grinding the plastic or a photo-cured material, in the three-dimensional printing device (30 of Fig. 1).

Referring to a portion of a cross-section of the phantom 32 customized to the patient, the inside of the phantom 32 customized to the patient is manufactured so as to include human body constituting materials that are substantially the similar as the actual anatomical structure of the patient. For example, models of the human body constituting materials such as a bone 620, a spinal code 630, or a lung 640 are manufactured inside the phantom 32.

As described above, the image reconstruction unit 120 reconstructs the three-dimensional phantom image 16 by reflecting pixel values of the medical images 22, and the inside of the phantom 32 customized to the patient is manufactured by reflecting a density of the actual bone 620, spinal code 630, or lung 640 of the patient, which is displayed on the medical images 22. In addition, the models of the human body constituting materials such as the bone 620, the spinal code 630, or the lung 640 may be manufactured so as to have different colors inside of the phantom 32 customized to the patient.

In addition, the inside of the phantom 32 customized to the patient is provided with a space into which a dosimeter (air ionization chamber) 611 or a dosimeter (film) 613 is inserted, the dosimeter (air ionization chamber) 611 or the dosimeter (film) 613 verifying or measuring the dose distribution of the radiation.

Furthermore, a setup mark 650 is marked outside of the phantom 32 customized to the patient, the setup mark 650 guiding the irradiation position of the radiation that is to be irradiated so as to verify or measure the dose distribution of the radiation.

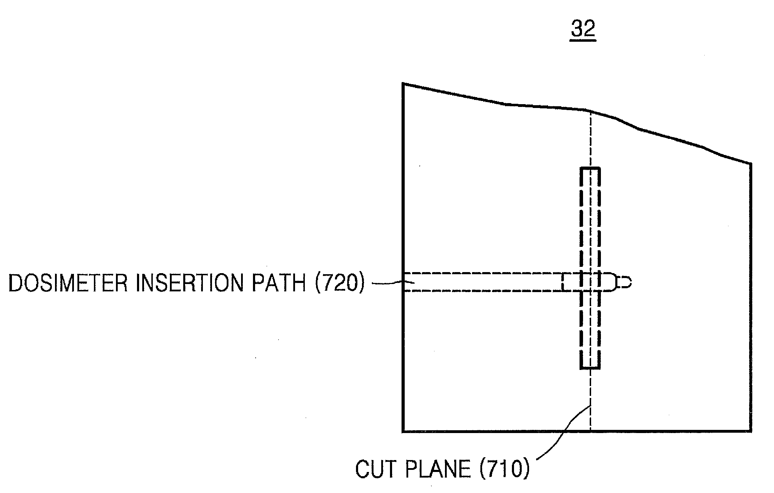

Fig. 7 is a side sectional view of the phantom 32 customized to the patient according to an exemplary embodiment of the present invention. Referring to Fig. 7, a side section of the phantom 32 customized to the patient is a drawing viewed from a side surface of the phantom 32 customized to the patient illustrated in Fig. 6.

As described above, the image reconstruction unit (120 of Fig. 1) reconstructs the three-dimensional phantom image (16 of Fig. 1) by including information about an insertion path 720 or cut plane 710 for inserting the dosimeter (air ionization chamber) 611 or the dosimeter (film) 613 into the phantom 32 customized to the patient.

After the phantom 32 customized to the patient is manufactured, the insertion path 720 may correspond to a space into which the dosimeter (air ionization chamber) 611 is to be inserted, the dosimeter (air ionization chamber) 611 verifying or measuring the dose distribution of the radiation. That is, the insertion path for inserting the dosimeter 611 (air ionization chamber) into the phantom 32 customized to the patient may be manufactured in an intaglio shape.

After the phantom 32 customized to the patient is manufactured, the cut plane 710 may be correspond to a cut plane that facilitates the insertion of the dosimeter (film) 613 for verifying or measuring the dose distribution of the radiation.

Referring again to Fig. 2, the storage unit 140 stores all pieces of information which is to be processed or is processed in the computing device 10.

For example, the storage unit 140 may pre-store the treatment plan information 12 and the information about the shape or the type of the dosimeter 14 and provides the stored treatment plan information and information of the dosimeter 14 to the image reconstruction unit 120 when the three-dimensional phantom image 16 is reconstructed. Here, the stored information of the dosimeter 14 may be commonly known 3D data (standard) stored in a library type.

In addition, the storage unit 140 stores information about the medical images 22 received from the medical image analysis device 20 and provides the stored information about the medical images 22 to the image reconstruction unit 120 when the three-dimensional phantom image 16 is reconstructed. Furthermore, the storage unit 140 may store the result of the three-dimensional phantom image 16 reconstructed in the image reconstruction unit 120.

The user interface unit 150 is hardware that includes a display device (not illustrated) providing information processed in the computing device 10 to the user and an input device such as a mouse or a keyboard, which receives a user input. The user interface unit 150 receives, from the user, user editing information about the internal and external structures of the patient's body or the space for the dosimeter 14, which are or is modeled from the medical images 22.

For example, the user may operate a keyboard operation or perform a mouse drag through the user interface unit 150 to insert the dosimeter into a position desired by the user in the three-dimensional phantom image 16 by using the information of the dosimeter 14, which is stored in the library type. The user may draw a new structure of the dosimeter 24 in person through the user interface unit 150 to insert the dosimeter 14 into the three-dimensional phantom image 16.

That is, the user interface unit 150 receives information in which the user wants to manually edit the three-dimensional phantom image 16 so as to meet user preference. At this time, the image reconstruction unit 120 may reconstruct the three-dimensional phantom image 16 by reflecting the editing information received in the user interface unit 150.

When the phantom 32 customized to the patient is manufactured in the three-dimensional printing device 30, the verification unit 160 verifies accuracy of the phantom 32 customized to the patient.

More specifically, after the dosimeter 14 is inserted into the manufactured phantom 32 customized to the patient, when the radiation is irradiated to the phantom 32 customized to the patient according to the treatment plan information 12, data about the dose distribution measured in the inserted dosimeter 14 is compared with the treatment plan information 12 to verify the accuracies of the phantom 32 customized to the patient ad the treatment plan information.

At this time, the verification result of the verification unit 160 may be provided in a graphic form by displaying an error on the three-dimensional phantom image 16 such that the user easily recognizes an error occurrence position through the user interface unit 150 but is not limited thereto.

As described above, the image reconstruction unit 120 corrects the three-dimensional phantom image 16 reconstructed according to the accuracies verified in the verification unit 160. The print data generation unit 130 regenerates the print data for three-dimensional printing, which is corrected on the basis of the corrected three-dimensional phantom image 16. When the three-dimensional printing device 30 remanufactures the phantom 32 customized to the patient corrected according to the regenerated print data for three-dimensional printing, the remanufactured phantom 32 customized to the patient may be manufactured which has a more accurate dose distribution compared to the phantom 32 customized to the patient manufactured before the verification is performed in the verification unit 160.

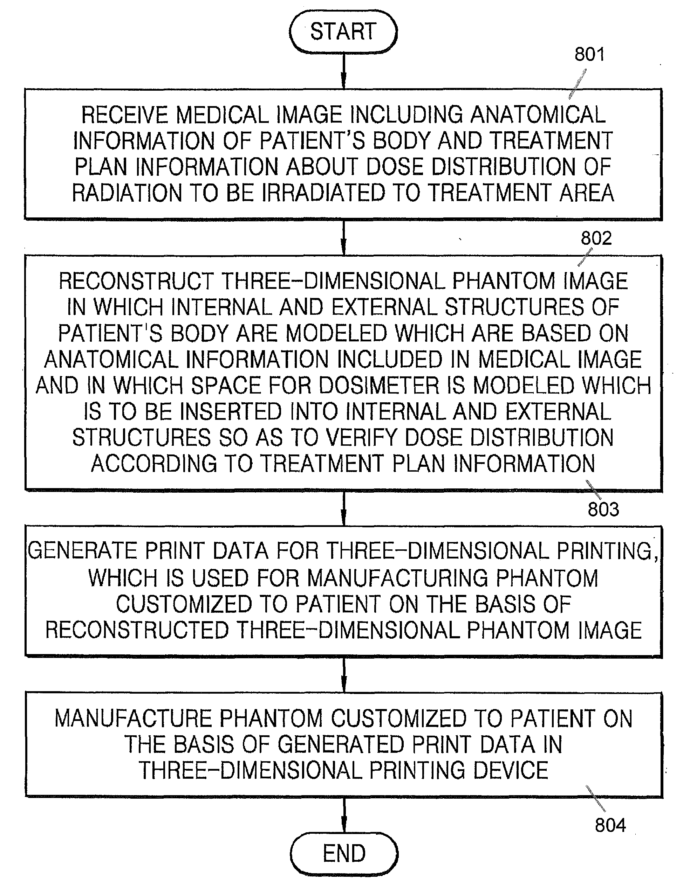

Fig. 8 is a flowchart of a method of manufacturing a phantom 32 customized to a patient, according to an exemplary embodiment of the present invention. Referring to Fig. 8, since the method of manufacturing the phantom 32 customized to the patient includes processes sequentially processed in the system 1 for manufacturing the phantom customized to the patient, even when description are omitted below, the descriptions provided above may also be applied to the method of manufacturing the phantom 32 customized to the patient of Fig. 8.

In operation 801, a data reception unit 110 receives medical images 22 that include anatomical information of a patient's body and treatment plan information 12 about a dose distribution of a radiation that is to be irradiated to a treatment area.

In operation 802, an image reconstruction unit 120 reconstructs a three-dimensional phantom image 16 in which internal and external structures of the patient's body are modeled which are based on the anatomical information and in which a space for a dosimeter 14 is modeled which is to be inserted into the internal and external structures so as to verify the dose distribution according to the treatment plan information 12.

In operation 803, a print data generation unit 130 generates print data for three-dimensional printing, which is used for manufacturing the phantom 32 customized to the patent on the basis of the reconstructed three-dimensional phantom image 16.

In operation 804, a three-dimensional printing device 30 manufactures the phantom 32 customized to the patent on the basis of the generated print data.

The embodiments of the present invention can be written as computer programs and can be implemented in general-use digital computers that execute the programs using a computer readable recording medium. In addition, structures of data used in the embodiments of the present invention can be recorded in computer readable medium through various means. Examples of the computer readable recording medium include magnetic storage media (e.g., ROM, floppy disks, hard disks, etc.) and optical recording media (e.g., CD-ROMs, or DVDs).

While this invention has been particularly shown and described with reference to preferred embodiments thereof, it will be understood by those skilled in the art that various changes in form and details may be made therein without departing from the spirit and scope of the invention as defined by the appended claims. The preferred embodiments should be considered in descriptive sense only and not for purposes of limitation. Therefore, the scope of the invention is defined not by the detailed description of the invention but by the appended claims, and all differences within the scope will be construed as being included in the present invention.

## Claims

1. An apparatus for generating print data about a phantom customized to a patent, the apparatus comprising:
a data reception unit that receives medical images including anatomical information of a patient's body and treatment plan information about a dose distribution of a radiation to be irradiated to a treatment area;
an image reconstruction unit that reconstructs a three-dimensional phantom image in which internal and external structures of the patient's body are modeled which are based on the anatomical information and in which a space for a dosimeter is modeled which is to be inserted into the internal and external structures so as to verify the dose distribution according to the treatment plan information; and
a print data generation unit that generates print data for three-dimensional printing, which is used for manufacturing the phantom customized to the patient on the basis of the reconstructed three-dimensional phantom image.

2. The apparatus of claim 1, wherein the image reconstruction unit reconstructs the three dimensional phantom image by modeling densities of materials constituting the internal structure by using pixel values of the received medical images.

3. The apparatus of claim 1, wherein the reconstructed three-dimensional phantom image includes information about an insertion path or cut plane for inserting the dosimeter into the phantom customized to the patient.

4. The apparatus of claim 1, wherein the reconstructed three-dimensional phantom image includes information about a mark which guides at least one of an irradiation position and a position of the dosimeter, which is used for verifying the dose distribution in the phantom customized to the patient.

5. The apparatus of claim 1, further comprising a verification unit that verifies accuracies of the phantom customized to the patient and the treatment plan information by comparing data about the dose distribution measured in the inserted dosimeter with the received treatment plan information when the radiation is irradiated to the phantom customized to the patient according to the treatment plan information after the phantom customized to the patient is manufactured on the basis of the generated print data, and the dosimeter is inserted into the phantom customized to the patient.

6. The apparatus of claim 5, wherein the image reconstruction unit corrects the reconstructed three-dimensional phantom image according to the verified accuracies, and the print data generation unit regenerates the print data for three-dimensional printing on the basis of the corrected three-dimensional phantom image.

7. The apparatus of claim 1, further comprising a user interface unit that receives, from a user, editing information about at least one of the modeled internal and external structures of the patient's body and the modeled space for the dosimeter, wherein the image reconstruction unit reconstructs the three-dimensional phantom image by modeling the at least one of the internal and external structures and the space according to the received editing information.

8. A method of manufacturing a phantom customized to a patent, the method comprising:
receiving medical images including anatomical information of a patient's body and treatment plan information about a dose distribution of a radiation to be irradiated to a treatment area;
reconstructing a three-dimensional phantom image in which internal and external structures of the patient's body are modeled which are based on the anatomical information and in which a space for a dosimeter 14 is modeled which is to be inserted into the internal and external structures so as to verify the dose distribution according to the treatment plan information;
generating print data for three-dimensional printing, which is used for manufacturing the phantom customized to the patient on the basis of the reconstructed three-dimensional phantom image; and
manufacturing the phantom customized to the patient on the basis of the generated print data in a three-dimensional printing device.

9. The method of claim 8, wherein the manufacturing manufactures the phantom customized to the patient by performing printing such that densities of materials constituting an inside of the phantom customized to the patient are different from one another according to pixels values of the received medical images.

10. The method of claim 8, wherein the measuring manufactures the phantom customized to the patient on the basis of the generated print data such that a space for the dosimeter is emptied inside of the phantom customized to the patient and a cut plane for inserting the dosimeter has a cut state.

11. A system for manufacturing a phantom customized to a patent, the system comprising:
a computing device that reconstructs a three-dimensional phantom image in which internal and external structures of a patient's body are modeled which are based on anatomical information included in medical images with respect to the patient's body and in which a space for a dosimeter is modeled which is to be inserted into the internal and external structures so as to verify a dose distribution according to treatment plan information about the dose distribution of a radiation to be irradiated to a treatment area, and generates print data for three-dimensional printing, which is used for manufacturing the phantom customized to the patient on the basis of the reconstructed three-dimensional phantom image; and
a three-dimensional printing device that manufactures the phantom customized to the patient on the basis of the generated print data.

12. The system of claim 11, wherein the computing device reconstructs the three-dimensional phantom image by modeling densities of materials constituting the internal structure by using pixel values of the received medical images.

13. The system of claim 11, wherein the reconstructed three-dimensional phantom image includes information about an insertion path or cut plane for inserting the dosimeter into the phantom customized to the patient.

14. The system of claim 11, wherein, after the phantom customized to the patient is manufactured by the three-dimensional printing device, and the dosimeter is inserted into the phantom customized to the patient, when the radiation is irradiated to the phantom customized to the patient according to the treatment plan information, the computing device verifies accuracies of the phantom customized to the patient and the treatment plan information by comparing data about the dose distribution measured in the inserted dosimeter with the received treatment plan information.

15. The system of claim 14, wherein the computing device corrects the reconstructed three-dimensional phantom image according to the verified accuracies and regenerates the print data for three-dimensional printing on the basis of the corrected three-dimensional phantom image, and the three-dimensional printing device manufactures the phantom customized to the patient on the basis of the regenerated print data.
